# EUROPEAN PATENT APPLICATION

(11) **EP 2 389 858 A2**
(43) Date of publication of application: **30.11.2011**
(21) Application number: 11166744.0
(22) Date of filing: 19.05.2011
(51) Int. Cl.: A61B 5/0205

(54) **Alarm generation method for patient monitoring, physiological monitoring apparatus, and computer program product for a physiological monitoring apparatus**

(30) Priority: 26.05.2010 US 787949
(71) Applicant: General Electric Company, Schenectady, NY 12345 (US)
(72) Inventor: Rantala, Borje, 00670, Helsinki (FI)
(74) Representative: Illingworth-Law, William Illingworth

(57) **Abstract**

A method for generating alarms in a physiological monitoring apparatus, a physiological monitoring apparatus, and a computer program product for a physiological monitoring apparatus are disclosed. A quality measure is calculated (23) for a physiological parameter, the quality measure being indicative of confidence level of the physiological parameter. An alarm is detected (22) when the physiological parameter reaches a predetermined alarm limit and the alarm is escalated in response to the detecting, wherein the escalating includes conditionally raising priority of the alarm. The escalation of the alarm is controlled (24) based on the quality measure. (FIG. 2)

## Description

### Background of the Invention

This disclosure relates generally to patient monitoring. More particularly, the present invention relates to escalation of alarms generated by physiological monitoring apparatuses, termed patient monitors below. Escalation here refers to a process that extends an alarm from a lower to a higher level of priority/severity, if the alarm persists and/or goes unacknowledged long enough.

Patient monitors are electronic devices designed to display physiological information about a subject. Electrocardiogram (ECG), electroencephalogram (EEG), plethysmographic signals, and signals related to blood pressure, temperature, and respiration represent typical physiological information contained in full-size patient monitors. Patient monitors are typically also furnished with alarming functionality to alert the nursing staff when a vital sign or physiological parameter of a patient exceeds or drops below a preset limit. Alarms are normally both audible and visual effects aiming to alert the staff to a life-threatening condition or to another event considered vital.

In addition to individual sensor/parameter alarms, patient monitors may be configured to raise combinatory alarms. That is, several physiological parameters may be used to determine a combined index and to give an alarm when the combined index fulfills a specific criterion. The combinatory alarms may range from simple combinations like "low heart rate and low arterial pressure" to complex rule-based scenarios used in various clinical support systems, for example. Below, the term physiological parameter is used to refer to the physiological variable to be monitored. As discussed above, the variable may be an individual parameter, such as heart rate or blood pressure, or a combinatory variable/index derived from multiple individual parameters. An individual physiological parameter may also represent a waveform signal value determined over a predefined period of time.

In most monitors, the alarm limits of a physiological parameter may be defined by the user, since the limits typically depend on patient etiology, age, gender, medication, and various other subjective factors. Each physiological parameter may also be assigned more than one alarm limit/criterion. That is, for a specific physiological parameter a patient monitor may raise alarms of different levels.

The monitor may also be provided with an alarm escalation mechanism to escalate unacknowledged and/or persistent alarms. Typically, an alarm is raised when the physiological parameter reaches a predefined low or high alarm limit, and the level of priority/severity of the alarm is increased if the alarm persists and/or remains unacknowledged for a predetermined period of time. The escalation rules stored in the monitor define how an alarm is escalated. Each alarm level may involve different alarming functionality.

In a clinical environment, the signals and parameters measured from a patient are prone to a wide variety of different interference sources. A pulse oximeter, for example, measures the Sp02 value from red and infrared waveforms through a complex algorithm. The plethysmographic signal often contains several types of noise, such as explicit optical and electrical interference from electrocautery and fluorescent ambient lighting, for example, and motion artifacts caused by movements of the patient. This makes alarm generation a demanding task, as the monitor should be both sensitive and specific in raising alarms. That is, the monitor should be able to recognize all true alarm events, without raising false or clinically irrelevant alarms. This task is further aggravated by the fact that many of the physiological signals measured from the patient are weak in nature and thus also extremely vulnerable to interference. Consequently, in a real clinical environment a large fraction of the alarms, even most alarms, may be false or at least clinically irrelevant. Such a large number of false or irrelevant alarms causes an enormous burden on the nursing staff and may also lead to impairment of the responses to true alarms.

### Brief Description of the Invention

The above-mentioned problem is addressed herein which will be comprehended from the following specification. In order to decrease the number of clinically irrelevant alarms, the escalation of a low priority alarm is controlled based on a quality measure indicative of the confidence level of the alarming parameter. Since the confidence level of the parameter may be lowered due to lowered quality of the respective physiological signal(s) or due to factors lowering the quality of the parameter determination process, the quality measure may be derived from the physiological signal(s) from which the parameter is derived and/or from the parameter determination process. The number of events of the physiological parameter only slightly crossing the lowest priority threshold is high during periods of lowered parameter confidence. Through quality based control of the alarm escalation the number of alarms caused by such events may be greatly reduced, since the control hampers the escalation of such alarms to higher priority alarms that would be clinically irrelevant.

In an embodiment, method for generating alarms in a physiological monitoring apparatus comprises calculating a quality measure for a physiological parameter, the quality measure being indicative of confidence level of the physiological parameter, wherein the physiological parameter is determined based on at least one physiological signal acquired from a subject. The method further includes detecting an alarm when the physiological parameter reaches a predetermined alarm limit, escalating the alarm in response to the detecting, wherein the escalating includes conditionally raising priority of the alarm, and controlling the escalating based on the quality measure.

In another embodiment, a physiological monitoring apparatus comprises a quality determination unit configured to calculate a quality measure for a physiological parameter, the quality measure being indicative of confidence level of the physiological parameter, wherein the physiological parameter is determined based on at least one physiological signal acquired from a subject. The apparatus further comprises an alarm detection unit configured to detect an alarm when the physiological parameter reaches a predetermined alarm limit, an alarm escalation unit configured to escalate the alarm and to conditionally raise priority of the alarm, and an escalation control unit configured to control escalation of the alarm based on the quality measure.

In a still further embodiment, a computer program product for a physiological monitoring apparatus comprises a first program product portion configured to control escalation of an alarm based on a quality measure determined for a physiological parameter derived from at least one physiological signal acquired by the apparatus from a subject, wherein the alarm relates to the physiological parameter and the quality measure is indicative of confidence level of the physiological parameter.

Various other features, objects, and advantages of the invention will be made apparent to those skilled in the art from the following detailed description and accompanying drawings.

### Brief Description of the Drawings

FIG. 1 illustrates one embodiment of an apparatus for monitoring patients,

FIG. 2 illustrates one embodiment of quality based control of alarm escalation;

FIG. 3 is a flow diagram illustrating another embodiment of the quality based control of alarm escalation;

FIG. 4, 5, and 6 illustrate an example of the effect of the quality measure on alarm escalation in case of a heart rate alarm;

FIG. 7 is a flow diagram illustrating one embodiment of escalation rate control; and

FIG. 8 illustrates an example of the functional entities of a patient monitor in terms of the escalation control.

### Detailed Description of the Invention

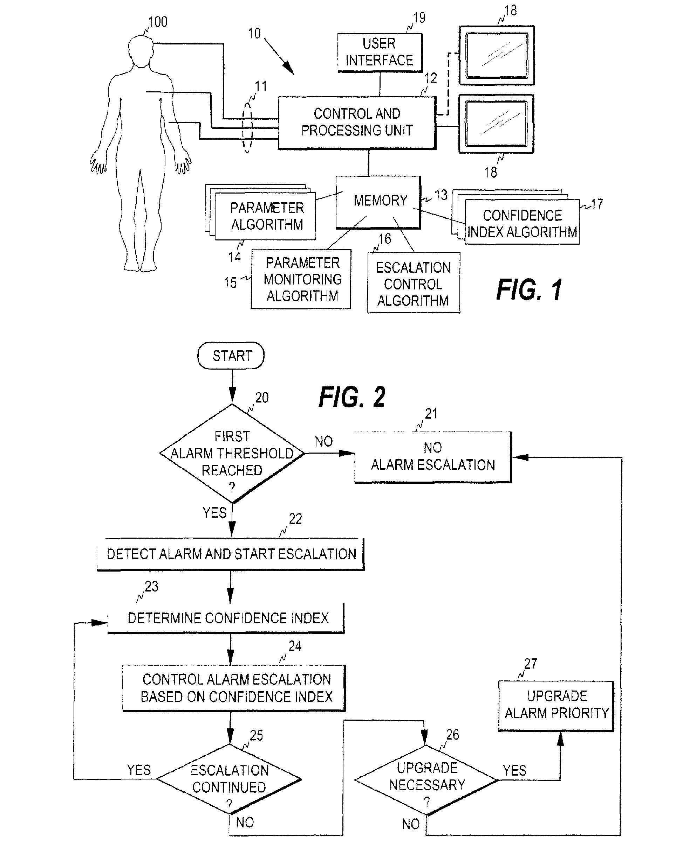

FIG. 1 illustrates one embodiment of a physiological monitoring apparatus/system 10 for monitoring a subject 100. A monitoring apparatus/system normally acquires a plurality of physiological signals 11 from the subject, where one physiological signal corresponds to one measurement channel. The physiological signals typically comprise several types of signals, such as ECG, EEG, blood pressure, respiration, and plethysmographic signals. Based on the raw real-time physiological signal data obtained from the subject, a plurality of physiological parameters may be determined, each physiological parameter being calculated from the waveform data of one or more of the physiological signals acquired from the subject. If a physiological parameter is derived from more than one physiological signal, i.e. from more than one measurement channel, the said physiological signals are usually of the same signal type. The physiological parameter may thus also represent a waveform signal value determined over a predefined period of time, although the physiological parameter is typically a distinct parameter derived from one or more measurement channels, such as heart rate derived from an ECG signal or an SP02 value derived from a plethysmographic signal.

The physiological signals 11 acquired from the subject are supplied to a control and processing unit 12 through a pre-processing stage (not shown) comprising typically an input amplifier and a filter, for example. The control and processing unit converts the signals into digitized format for each measurement channel. The digitized signal data may then be stored in the memory 13 of the control and processing unit. The digitized signal data is utilized by parameter algorithms 14 adapted to record, when executed by the control and processing unit, the time series of the physiological parameters to be monitored. The obtained time series of the physiological parameters may be stored in the memory. Generally, each physiological parameter may be assigned one or more alarm limits to alert the nursing staff when the parameter reaches or crosses the alarm limit. It is assumed here that at least one physiological parameter is assigned multiple alarm levels of increasing priority/severity, and that alarm escalation is used to escalate a low level alarm to the next level of priority/severity, if the low level alarm persists and/or goes unacknowledged long enough. The monitoring and alarm escalation of one such physiological parameter is discussed below.

The control and processing unit uses a parameter monitoring algorithm 15 to monitor the successive values of the parameter. This typically involves comparison of the parameter values with at least one alarm limit to detect whether an alarm is to be raised. When a crossing of an alarm limit is detected, the control and processing unit may or may not inform the user of the alarm. In response to the detection of the alarm limit crossing, the control and processing unit starts to escalate the alarm by an escalation control algorithm 16 that controls the escalation.

For obtaining input data for the escalation control, the control and processing unit further uses one or more confidence index algorithms 17 to determine a quality measure indicative of the confidence level of the monitored physiological parameter. The quality measure is termed confidence index in this context, since it reflects the confidence level (i.e. reliability) of the monitored parameter. Since the confidence level of the parameter depends on signal quality, the measure may be derived from the physiological signal(s) based on which the monitored physiological parameter is determined. However, the confidence level of the monitored parameter may also be lowered even though the quality of the physiological signal is high. Therefore, the confidence index may also be obtained from the parameter determination process. The confidence index may also be determined as a combination of a first measure indicative of the quality of the physiological signal(s) and a second measure indicative of the quality/reliability of the parameter. Consequently, the confidence index may reflect the confidence level through the quality of the physiological signal(s), through the quality of the parameter determination process, or through both the quality of the physiological signal(s) and the quality of the parameter determination process.

The confidence index algorithms may also be integrated with the parameter algorithms, especially if the confidence index is calculated in connection with the parameter determination and/or if the confidence index is determined substantially continuously similarly as the parameter. Each integrated algorithm then outputs the time series of both the parameter and the associated confidence index.

The control and processing unit is further configured to control the display unit 18 of the apparatus. A display control algorithm may be stored in the memory of the control and processing unit and the apparatus may be provided with more than one display unit. The user may supply information and control the apparatus/system through user interface 19. In addition to the visual and audible effects related to each alarm level, an alarm may be transmitted to an external monitoring unit through a network, for example.

FIG. 2 is a flow diagram illustrating one embodiment of the operation of the control and processing unit for controlling the escalation of a low level alarm. The control and processing unit monitors the values of the physiological parameter and examines whether the values reach the first alarm limit, i.e. the first parameter value at which an alarm is detected (step 20). Since no alarm is detected before that, no escalation is used until the physiological parameter crosses the first alarm limit (cf. step 21). When the control and processing unit detects that the first alarm limit is reached, an alarm escalation process is started (step 22). Since the priority of the alarm is low, it may be indicated in a non-disturbing manner at the bedside or at another site where a caregiver may easily acknowledge it. It is also possible that no indication of the low priority alarm is given to the caregiver, but the caregiver is informed of an alarm only later when the priority of the alarm is increased, cf. step 27. The lowest priority alarm event may thus be hidden.

In response to the start of the alarm escalation, the control and processing unit starts to determine the confidence index (step 23). The determination may be carried out at regular intervals, such as every 1-2 seconds. The control and processing unit further starts to control the escalation based on the confidence index (step 24). The quality based control carried out in step 24 ends when the process detects that there is no more a need to escalate the alarm (the alarm goes out or is acknowledged), cf. steps 25 and 26/no. The quality based control of step 24 may also end when the priority of the alarm is to be increased (steps 25, 26/yes, and 27). However, a new quality based escalation process may also be started upon increase of the priority. That is, the quality based control of the escalation may be applied to one or more priority levels.

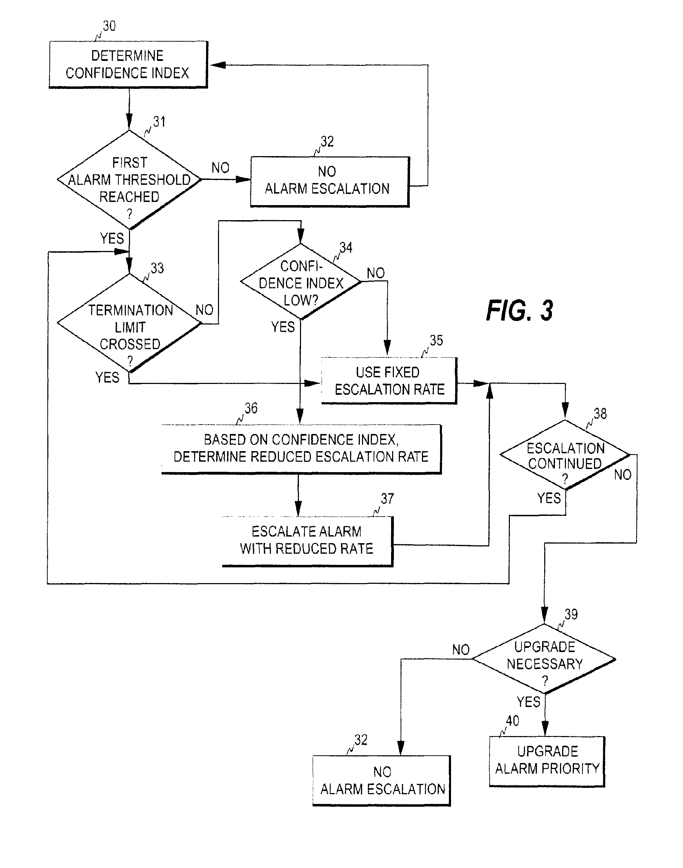

FIG. 3 illustrates one embodiment of the actual escalation control. In this embodiment, the confidence index is measured substantially continuously, regardless of whether or not escalation is in progress (step 30). Steps 31 and 32 correspond respectively to steps 20 and 21 of FIG. 2. Depending on the confidence index, a reduced escalation rate is used when the value of the parameter is between the first alarm limit and a predetermined termination limit that defines the parameter level above which the escalation rate is no more reduced. Thus, the reduction of the escalation rate is carried out only when the physiological parameter is between the first (low priority) limit and the termination limit, provided that the confidence index meets predetermined criteria concurrently. To carry out the rate reduction, the control and processing unit first examines in steps 31 and 33 whether the current value of the parameter is between the first limit and the termination limit. If this is the case (step 33/no), the control and processing unit further examines the current value of the confidence index (step 34). If the current value has dropped from its normal high value, the control and processing unit uses reduced escalation rate, which lengthens the escalation time, i.e. the time period elapsing from the low level alarm to the priority upgrade. The reduced escalation rate is determined based on the confidence index in step 36. If the confidence index is in its normal high value (or value range), the control and processing unit uses fixed escalation rate (step 35). That is, the next priority level will be reached after a fixed time period, provided that the alarm persists and the signal/parameter quality (confidence index) remains high for the entire period. This corresponds to conventional escalation, where two successive priority levels are a fixed time period apart. Steps 38-40 correspond to steps 25-27 of FIG. 2, respectively. The reduction of the escalation rate thus here refers to reduction as compared to the fixed escalation rate.

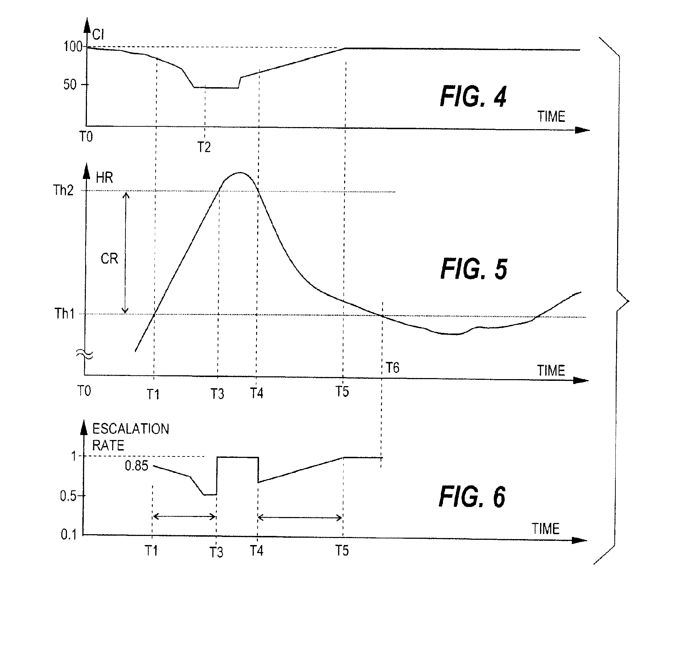

FIGs. 4 to 6 illustrate an example of the escalation control of FIG. 3 by showing an example of the behavior of the escalation rate during variation of the parameter confidence level. It is assumed here that the physiological parameter measured from the patient is heart rate. FIG. 4 shows the confidence index (CI), FIG. 5 the heart rate (HR), and FIG. 6 the escalation rate as a function of time. At time instant T0, the confidence index begins to drop from its normal value, which is 100 in this example. However, since the heart rate has not yet reached the first alarm limit (Th1), no alarm escalation is applied, cf. step 32 of FIG. 3. At time instant T1, the heart rate reaches the first alarm limit. In response to this, an alarm event of the lowest priority is detected and the escalation of the alarm starts. Since the CI value is at this time instant slightly below its normal value, the escalation rate to be applied is also slightly lower than the normal value. In this example, the value of CI is at this time instant 85, and the escalation rate is 0.85, while the fixed (normal) rate is 1 (units per second). If the CI values may vary between 100 and 0, the escalation rate may vary, according to the CI variations, between 1 and 0.1, for example. The signal quality algorithm may be such that the index value maintains at 100 as long as the signal/parameter quality exceeds a predefined level, but begins to drop gradually as the quality drops below the level.

Between time instants T1 and T3, the escalation rate is directly proportional to the CI value (escalation rate = CI(t) x 0.01). However, at time instant T3, the heart rate reaches the termination limit Th2. In response to this, the quality-based control of the escalation rate is terminated and the escalation control starts to use the fixed escalation rate of 1 units per second, even though the concurrent CI value is only 50, cf. steps 33/yes and 35 of FIG. 3. The escalation rate remains at this fixed value as long as the heart rate remains above the termination limit. At time instant T4, the heart rate drops below the termination limit, and the quality-based control of the escalation rate resumes. In response to this, the escalation rate drops to the value of CI(t=T4) x 0.01, where CI(t=T4) is the value of the confidence index at t=T4.

At time instant T5, the confidence index again reaches its normal level and maintains at the said high level henceforward. Therefore, fixed escalation rate is applied from T5 onwards, if the next priority level is not reached yet. At time instant T6, the heart rate drops below the limit of the lowest priority alarm and the escalation of the alarm is stopped, if it is still in progress. It is to be noted here that the escalation rate of FIG. 6 is applied to the original lowest priority alarm detected at time instant T1 and that the next priority level may be reached at any time instant between T1 and T6. A new escalation process may be started when the said next priority level is reached.

The control of the escalation may be implemented, for example, by incrementing a counter at a rate or value that depends on the confidence index and the parameter in the above-described manner.

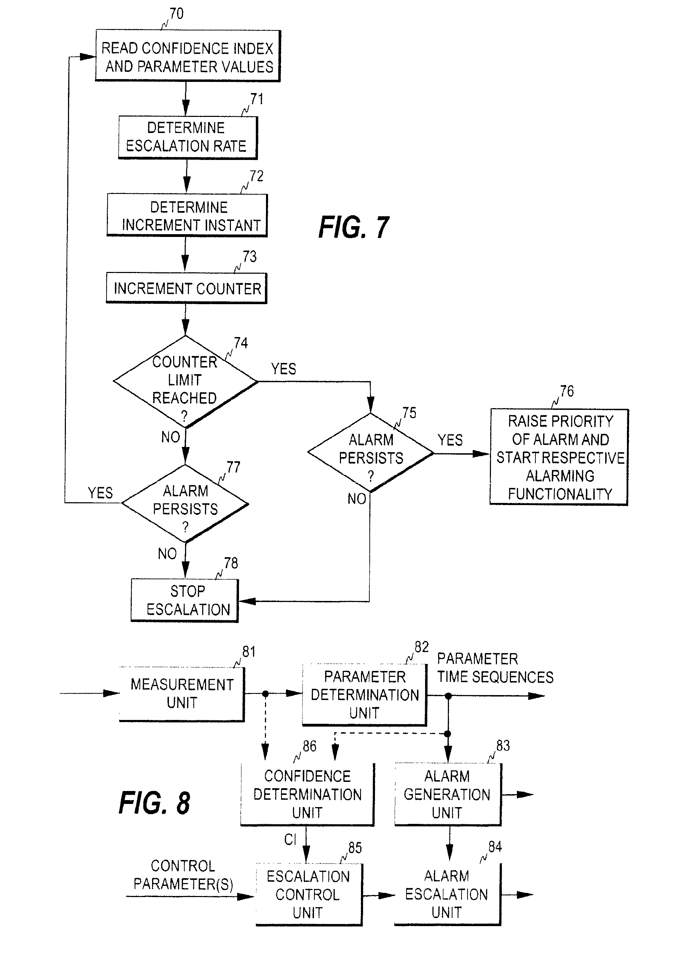

An embodiment of counter-based control is illustrated in FIG. 7. Upon start of the escalation, the control and processing unit reads the values of the confidence index and the physiological parameter and determines the escalation rate based on the said values (steps 70, 71). Based on the escalation rate, the control and processing unit further determines the next increment instant (step 72). It is assumed here that the counter is incremented at intervals of 10 ms or at multiples thereof, where the interval of 10 ms corresponds to the fixed rate. Thus, when the signal and/or parameter quality is high, or when the parameter is beyond the termination limit, the control and processing unit increments the counter by 100 in each second. When the confidence index is 0.1 or below, the counter is incremented at intervals of 100 ms, i.e. escalation is delayed by a factor of 10.

Having incremented the counter in step 73, the control and processing unit examines whether the counter limit has been reached (step 74). If this is the case and the alarm still persists, the priority of the alarm is increased and the alarming functions associated with the new priority level are triggered (step 76). If the control and processing unit detects, upon update of the counter, that the alarm does not persist any more, the escalation process is stopped (steps 75, 77 and 78). If the counter limit has not yet been reached and the alarm still persists, the process returns to step 70 to carry out a new counter update. Instead of incrementing the counter by one at a variable rate, the counter may be incremented at fixed rate by an increment value depending on the confidence index and the parameter in the above-described manner.

The counter limit for the priority level raise may be, for example, 700. That is, if the signal quality is continuously high, the next priority level will be reached in 7 seconds. Between time instants T2 and T3, for example, the control and processing unit increments the counter at a rate of 50 per second, since the concurrent CI value is 50 and the example assumes that escalation rate is proportional to the current CI value. However, in this example the escalation rate also has a minimum value of 0.1, which is reached when the CI value drops to 10. Therefore, if the CI is below 10, the escalation proceeds at a rate which is one tenth of the (normal) fixed rate of step 35. In other words, the maximum time period between the successive priority levels is ten-fold compared to the full escalation rate of step 35. In the above example, the total escalation time may thus vary between 7 and 70 seconds.

In the above examples, the escalation of the alarm is thus "delayed" between time instants T1 and T3, and also between time instants T4 and T5, if the counter does not reach the limit of the next priority level before time instant T4. The delay here refers to the lengthening of the escalation time, compared to the traditional escalation using the same fixed escalation rate as in step 35 (or to the situation in which the confidence index keeps the escalation rate at its maximum for the entire escalation time). The new escalation process that starts from the priority upgrade may be carried out without delaying, since the alarm is now of higher priority. In other words, the delay mechanism may be applied to the escalation of the lowest priority alarms only.

In the above embodiments, the reduction of the escalation rate is stopped if the physiological parameter reaches the termination limit. Consequently, the control range (CR, FIG. 5) between the lowest priority alarm limit and the termination limit determines the escalation control area, where escalation is controlled based on signal and/or parameter quality. By controlling the distance between the said two limits, the extent of the "delaying" may therefore be controlled. The monitor may be provided with a user-controllable parameter, termed acuity parameter in this context, which controls the alarm sensitivity of the monitor, i.e. the allowable extent of the escalation control. If the monitor is used in an Intensive Care Unit, for example, the user may select maximum value of the acuity parameter, thereby to maximize the "escalation efficiency" of the monitor. This value of the acuity parameter may reduce the control range CR to zero. If, however, the monitor is in a ward of low risk patients (lower acuity), the "escalation efficiency" may be reduced by selecting a lower value of the acuity parameter, thereby to reduce the number of nuisance alarms.

The control and processing unit may determine the termination limit Th2 based on the acuity parameter and the low priority limit Th1. The determination may be carried out, for example, as follows: Th2 = P x Th1 + F, where the values of P and F depend on the acuity parameter selected. The maximum of the acuity parameter may correspond to P=1 and F=0, for example, as indicated above. The user may select the acuity parameter from two or more alternatives, each alternative being assigned dedicated values of P and F. The only limit that needs to be controlled by the user is therefore the low priority limit Th 1. A single control parameter, the acuity parameter, may be used to control the termination limits of multiple physiological parameters to be monitored.

It is also possible that the control and processing unit adjusts the control range at each priority rise, thereby to increase the "escalation efficiency" as the priority of the alarm increases. At the same time, the counter limit value may be adjusted.

In terms of the escalation control, the functionalities of the control and processing unit 12 may be divided into the units shown in FIG. 8. A measurement unit 81 is configured to measure the physiological signal data from the subject. A parameter determination unit 82 is configured to determine, based on at least one physiological signal, the physiological parameter(s) to be monitored. An alarm generation unit 83 is configured to detect an alarm event and optionally produce an alarm when the monitored physiological parameter reaches a predetermined alarm limit. An alarm escalation unit 84 is configured to escalate the detected alarm to the next level of priority according to any of the above embodiments, and an escalation control unit 85 is configured to control the escalation of the alarm based on a quality measure that may be derived by a confidence determination unit 86 from the physiological signal(s), from the parameter determination process, or from both the physiological signal(s) and the parameter determination. It is to be noted that FIG. 8 illustrates the division of the functionalities of the control and processing unit in logical sense and in view of the alarm generation and escalation. In a real apparatus the functionalities may be distributed in different ways between the elements or units of the apparatus. For example, the functions of the confidence determination unit 86 may be divided between several hardware units of the apparatus by defining a plurality of quality parameters in various parts of the device and determining the final confidence index based on the quality parameters. For example, quality parameters may be determined that are indicative of sensor attachment ("leads-almost-off"), noise/high frequency interference, presence/absence of a defibrillator, 50/60 Hz line interference, and motion artefacts. The first three parameters may be determined in the data acquisition module of the front-end, while the rest may be determined in a noise rejection filter and in the actual parameter algorithm. When interference/artefacts are removed, the amount of interference/artefacts removed is also known and can be used to derive a value for the quality parameter. The control and processing unit may thus determine the final confidence index based on the quality parameters determined in various parts of the apparatus. The apparatus may also be implemented as an auxiliary monitor utilizing the physiological data collected by another device. Units 81 and 82 may therefore belong to another device to which the auxiliary monitor is connectable.

As discussed above, the confidence index may be determined based on a first measure indicative of the quality of the physiological signal(s) or based on a second measure indicative of the quality/reliability of the physiological parameter, or based on a combination of the first and second measures. It is obvious that the quality of a physiological signal affects the confidence level of a parameter derived from the said signal. However, the confidence level of the physiological parameter may also be lowered even though the quality of the physiological signal is high. This is the case if certain factors of uncertainty are detected in the parameter determination process, which lower the reliability of the determined parameter. These factors are mainly patient originated physiological factors, such as various cardiac arrhythmias that decrease the accuracy of the determination of a cardiac parameter, such as heart rate. The presence of these factors may be monitored in the parameter determination process by monitoring variations of certain variables in the parameter determination process, such as R-R variations in the determination of heart rate. Detected presence of such factors may then be used to decrease the value of the quality measure. The set of resources that may be used to calculate the quality measure thus include the physiological signal(s) and/or the parameter determination process.

The above mechanism may be applied to all physiological parameters to be monitored, with the exception of a few parameters that are critical (immediate life risk) and insensitive to artefacts (no artefact originated false alarms). Such parameters/events are, for example, 02, apnea, and asystole. The fixed escalation rate of step 35 may be different for each physiological parameter to which the escalation control is applied. However, a given quality drop may cause the same relative drop in the escalation rate of each parameter for which the escalation rate is controlled.

A conventional patient monitor may be upgraded to enable the monitor to control the alarm escalation in the above-described manner. Such an apparatus upgrade may be implemented, for example, by delivering to the monitor a software module that may contain the above functionality. The software module may be delivered, for example, on a data carrier, such as a CD or a memory card, or through a telecommunications network. However, since the software module may utilize the parameters already determined by the patient monitor and since the monitor may also determine a quality measure indicative of the confidence level of the monitored parameter, the software module does not necessarily comprise more than a program product portion configured to control the escalation of the alarm based on the quality measure. Thus, software module may only add the quality-based control feature to existing software that uses fixed escalation rate. This may be implemented, for example, through a minor update of the entity that increments the counter, i.e. by updating the counter increment logic. However, the software module may also determine the confidence index or other parameters not readily available in the monitor. In addition to the quality-based escalation control functionality, the software module may thus comprise any of the functionalities of the units of FIG. 8.

This written description uses examples to disclose the invention, including the best mode, and also to enable any person skilled in the art to make and use the invention. The patentable scope of the invention is defined by the claims, and may include other examples that occur to those skilled in the art. Such other examples are intended to be within the scope of the claims if they have structural or operational elements that do not differ from the literal language of the claims, or if they have structural or operational elements with insubstantial differences from the literal language of the claims.

Various aspects and embodiments of the present invention are defined by the following numbered clauses:
1. A method for generating alarms in a physiological monitoring apparatus, the method comprising:
   - calculating a quality measure for a physiological parameter, the quality measure being indicative of confidence level of the physiological parameter, wherein the physiological parameter is determined based on at least one physiological signal acquired from a subject;
   - detecting an alarm when the physiological parameter reaches a predetermined alarm limit;
   - escalating the alarm in response to the detecting, wherein the escalating includes conditionally raising priority of the alarm; and
   - controlling the escalating based on the quality measure.
2. The method according to clause 1, wherein the calculating includes calculating the quality measure based on at least one resource set in a group of resource sets including a first resource set of the at least one physiological signal and a second resource set comprising determination process of the physiological parameter.
3. The method according to clause 1 or clause 2, wherein the controlling includes
   - using a reduced escalation rate for escalation of the alarm when the quality measure indicates a reduction in the confidence level; and
   - using a non-reduced escalation rate for escalation of the alarm when the quality measure indicates that the confidence level is devoid of a reduction.
4. The method according to any preceding clause, wherein the controlling further includes stopping use of the reduced escalation rate and taking the non-reduced escalation rate into use when the physiological parameter crosses a predetermined termination limit.
5. The method according to any preceding clause, wherein the using the reduced escalation rate includes reducing the escalation rate proportionally to the quality measure.
6. The method according to any preceding clause, wherein in the controlling further comprises stopping the using the reduced escalation rate when the quality measure reaches a predetermined lower limit.
7. The method according to any preceding clause, further comprising adjusting the termination limit based on a control parameter representing a general acuity level of the subject, wherein the adjusting includes calculating the termination limit based on the predetermined alarm limit and the control parameter.
8. The method according to any preceding clause, further comprising informing a caregiver of the alarm, wherein the alarm is a low priority alarm
9. The method according to any preceding clause, wherein the controlling includes adjusting increment rate of an escalation counter based on the quality measure.
10. A physiological monitoring apparatus comprising:
   - a confidence determination unit configured to calculate a quality measure for a physiological parameter, the quality measure being indicative of confidence level of the physiological parameter, wherein the physiological parameter is determined based on at least one physiological signal acquired from a subject;
   - an alarm detection unit configured to detect an alarm when the physiological parameter reaches a predetermined alarm limit;
   - an alarm escalation unit configured to escalate the alarm and to conditionally raise priority of the alarm; and
   - an escalation control unit configured to control escalation of the alarm based on the quality measure.
11. The apparatus according to clause 10, wherein the quality measure is calculated based on at least one resource set in a group of resource sets including a first resource set of the at least one physiological signal and a second resource set comprising determination process of the physiological parameter.
12. The apparatus according to clause 10 or clause 11, wherein the escalation control unit is configured to use a reduced escalation rate for the escalation when the quality measure indicates a reduction in the confidence level and to use a non-reduced escalation rate for the escalation when the quality measure indicates that the confidence level is devoid of a reduction.
13. The apparatus according to any of clauses 10 to 12, wherein the escalation control unit is further configured to stop reduction of the escalation rate and take a non-reduced escalation rate into use when the physiological parameter crosses a predetermined termination limit.
14. The apparatus according to any of clauses 10 to 13, wherein the escalation control unit is further configured to reduce the escalation rate proportionally to the quality measure.
15. The apparatus according to any of clauses 10 to 14, wherein the escalation control unit is further configured to stop reduction of the escalation rate when the quality measure reaches a predetermined lower limit.
16. The apparatus according to any of clauses 10 to 15, wherein the termination limit is calculated based on the predetermined alarm limit and a control parameter representing a general acuity level of the subject.
17. The apparatus according to any of clauses 10 to 16, wherein the alarm detection unit is further configured to inform a caregiver of the alarm, wherein the alarm is a low priority alarm
18. The apparatus according to any of clauses 10 to 17, wherein the escalation control unit is configured to adjust increment rate of an escalation counter based on the quality measure.
19. A computer program product for a physiological monitoring apparatus, the computer program product comprising a first program product portion configured to control escalation of an alarm based on a quality measure determined for a physiological parameter derived from at least one physiological signal acquired by the apparatus from a subject, wherein the alarm relates to the physiological parameter and the quality measure is indicative of confidence level of the physiological parameter.
20. The computer program product according to clause 19, further comprising a second program product portion configured to determine the quality measure.

## Claims

1. A method for generating alarms in a physiological monitoring apparatus, the method comprising:
- calculating (23; 30) a quality measure for a physiological parameter, the quality measure being indicative of confidence level of the physiological parameter, wherein the physiological parameter is determined based on at least one physiological signal (11) acquired from a subject (100);
- detecting (22; 31) an alarm when the physiological parameter reaches a predetermined alarm limit;
- escalating the alarm in response to the detecting, wherein the escalating includes conditionally raising priority of the alarm; and
- controlling (24; 35-37) the escalating based on the quality measure.

2. The method according to claim 1, wherein the calculating includes calculating the quality measure based on at least one resource set in a group of resource sets including a first resource set of the at least one physiological signal (11) and a second resource set comprising determination process of the physiological parameter.

3. The method according to claim 1 or claim 2, wherein the controlling includes
- using (37) a reduced escalation rate for escalation of the alarm when the quality measure indicates a reduction in the confidence level; and
- using (35) a non-reduced escalation rate for escalation of the alarm when the quality measure indicates that the confidence level is devoid of a reduction.

4. The method according to any preceding claim, wherein the controlling further includes stopping use of the reduced escalation rate and taking the non-reduced escalation rate into use when the physiological parameter crosses a predetermined termination limit (Th2).

5. The method according to any preceding claim, wherein the using the reduced escalation rate includes reducing the escalation rate proportionally to the quality measure.

6. The method according to any preceding claim, wherein in the controlling further comprises stopping the using the reduced escalation rate when the quality measure reaches a predetermined lower limit.

7. The method according to any preceding claim, wherein the controlling includes adjusting (71-73) increment rate of an escalation counter based on the quality measure.

8. A physiological monitoring apparatus comprising:
- a confidence determination unit (12, 17; 86) configured to calculate a quality measure for a physiological parameter, the quality measure being indicative of confidence level of the physiological parameter, wherein the physiological parameter is determined based on at least one physiological signal (11) acquired from a subject (100);
- an alarm detection unit (12, 15; 83) configured to detect an alarm when the physiological parameter reaches a predetermined alarm limit;
- an alarm escalation unit (12; 84) configured to escalate the alarm and to conditionally raise priority of the alarm; and
- an escalation control unit (12, 16; 85) configured to control escalation of the alarm based on the quality measure.

9. The apparatus according to claim 8, wherein the quality measure is calculated based on at least one resource set in a group of resource sets including a first resource set of the at least one physiological signal (11) and a second resource set comprising determination process of the physiological parameter.

10. The apparatus according to claim 8 or claim 9, wherein the escalation control unit is configured to use a reduced escalation rate for the escalation when the quality measure indicates a reduction in the confidence level and to use a non-reduced escalation rate for the escalation when the quality measure indicates that the confidence level is devoid of a reduction.

11. The apparatus according to any of claims 8 to 10, wherein the escalation control unit (12, 16; 85) is further configured to stop reduction of the escalation rate and take a non-reduced escalation rate into use when the physiological parameter crosses a predetermined termination limit.

12. The apparatus according to any of claims 8 to 11, wherein the escalation control unit (12, 16; 85) is further configured to stop reduction of the escalation rate when the quality measure reaches a predetermined lower limit.

13. The apparatus according to any of claims 8 to 12, wherein the termination limit is calculated based on the predetermined alarm limit and a control parameter representing a general acuity level of the subject (100).

14. The apparatus according to any of claims 8 to 13, wherein the alarm detection unit (12, 15; 83) is further configured to inform a caregiver of the alarm, wherein the alarm is a low priority alarm.

15. A computer program product for a physiological monitoring apparatus, the computer program product comprising a first program product portion configured to control escalation of an alarm based on a quality measure determined for a physiological parameter derived from at least one physiological signal (11) acquired by the apparatus from a subject (100), wherein the alarm relates to the physiological parameter and the quality measure is indicative of confidence level of the physiological parameter.
